# FASCICULE DE BREVET EUROPEEN

(11) **EP 0 615 449 B1**
(45) Date de publication et mention de la délivrance du brevet: **15.09.1999**
(21) Numéro de dépôt: 93921977.0
(22) Date de dépôt: 01.10.1993
(51) Int. Cl.: A61K 31/525, A61F 6/04, A61B 19/04

(54) **UTILISATION DE DERIVES DE LA FLAVINE POUR LA REALISATION D'ARTICLES D'HYGIENE MEDICO-CHIRURGICAUX ET LES ARTICLES D'HYGIENE AINSI REALISES**
VERWENDUNG VON FLAVIN-DERIVATEN ZUR HERSTELLUNG VON MEDIZINISCH-CHIRUGISCHEN HYGIENEARTIKELN UND DIE SO ERHALTENEN HYGIENEARTIKEL
USE OF FLAVINE DERIVATIVES IN THE PRODUCTION OF MEDICO-SURGICAL SANITARY ARTICLES AND SANITARY ARTICLES SO OBTAINED

(30) Priorité: 02.10.1992 FR 9212180
(43) Date de publication de la demande: 21.09.1994
(73) Titulaire: BERQUE, Jean, F-16100 Cognac (FR)
(72) Inventeur: BERQUE, Jean, F-16100 Cognac (FR)
(74) Mandataire: Burtin, Jean-François
(86) Numéro de dépôt international: FR9300962
(87) Numéro de publication internationale: WO9407499

(56) Documents cités:
- EP-A- 0 355 245
- WO-A-92/17173

## Description

La présente invention est destinée à améliorer la protection des préservatifs, gants médico-chirurgicaux et doigtiers par l'adjonction, lors de leur fabrication, d'un principe actif coloré, principalement de la vitamine B2 et du FAD dont les propriétés biophysiques en matière de coloration, et biochimiques font de ces molécules des agents antiviraux biologiques efficaces et atoxiques. En plus de l'intérêt de cette application vis-à-vis du Sida et de l'Herpès, les propriétés de ces deux molécules indiquent qu'elles peuvent également renforcer la protection anti-bactérienne.

On sait que les préservatifs sont utilisés parfois dans un but contraceptif mais le plus souvent dans un but hygiénique pour la prévention des maladies sexuellement transmissibles et, en particulier, de l'Herpès génital et du Sida. De la même façon, les gants médico-chirurgicaux stériles et à usage unique ou les doigtiers, ont pour fonction principale d'éviter une contamination entre soignants et patients.

Au vu des recherches du Demandeur et des découvertes concernant la Riboflavine et, en particulier, ses propriétés anti-virales (HSV1, V2, HIV1 et HIV2), il est apparu qu'éventuellement l'inclusion de Vitamine B2 ou d'autres produits biologiques, dans le latex ou dans le chlorure de polyvinyle, permettrait d'en renforcer les fonctions isolantes et par la même, de limiter au maximum le risque de propagation virale et présente en outre des propriétés anti-allergique des plus intéressantes.

La technique actuelle utilise des préservatifs et des gants médico-chirurgicaux le plus souvent translucides et non opaques aux U.V, ces articles sont en général en latex ou en chlorure de polyvinyle.

Des produits colorés ont été déjà commercialisés dans un but essentiellement distinctif ou d'originalité. Certains procédés adjoignant des antiseptiques tels que les sels d'ammoniums quaternaires sont à l'étude ou sont déjà utilisés. On a déjà également adjoint aux gants médicaux ou aux préservatifs des agents inhibant la Transcriptase Inverse.

Les recherches théoriques du Demandeur concernant la photosynthèse protéique à l'échelle animale et la photosynthèse virale, ont amené à constater et à démontrer les propriétés anti-virales de la Riboflavine et de son dérivé actif le FAD, et accessoirement, de la Vitamine PP et de son métabolite actif le NADP. Cette activité anti-virale recherchée au départ en fonction de son absorption des rayons lumineux et des propriétés biologiques de la Riboflavine, est pleinement corroborée par la formule chimique du FAD. L'association d'une base nucléique, l'Adénine, et d'un noyau ribose doublement phosphorylé fait du FAD, une molécule diphosphonucléotidique dont on connait les effets inhibiteurs sur la Transcriptase Inverse. Le FAD présente un double avantage par rapport aux molécules synthétiques déjà utilisées contre l'Herpès et le Sida; c'est tout d'abord la présence de la Flavine en complément de l'Adénine et du ribose diphosphorylé. Les propriétés d'absorption de la lumière de la Flavine ainsi que son affinité pour la lumière UV paraissent d'une grande importance, dans le sens que le noyau Flavine, dénommé chimiquement reste iso-alloxazine, en localisant le rayonnement UV sur la molécule de FAD permet vraisemblablement d'accroître l'efficacité de la partie diphosphonucléotidique. On sait en outre que les Flavoprotéines, enzymes majeurs de l'oxydo-réduction sont trichromes. Cette propriété qui pourrait paraitre une constatation usuelle pour un biochimiste, semble d'un grand intérêt au niveau de la photosynthèse protéique et des messages opto-électroniques parvenant aux cellules et plus particulièrement à l'ADN nucléaire.

L'étude structurelle du FAD apporte une confirmation dans la conception de son rôle d'émetteur-récepteur "son et lumière"; tout d'abord, le triple noyau iso-alloxazine incite à penser qu'il se comporte mon seulement comme un miroir à trois faces, mais aussi comme un émetteur-récepteur radio avec deux haut-parleurs stéréophoniques. La molécule de FAD pourrait bien correspondre à une molécule laser couplée à un système émetteur-récepteur à modulation variable. Les deux noyaux de l'Adénine portent chacun deux atomes d'azote ainsi que les deux noyaux du reste iso-alloxazine. Ils peuvent donc s'apparenter aux composés azoïques dont on connait l'utilisation dans l'industrie des colorants. Une telle structure chimique n'est pas sans évoquer également la diazocopie, c'est-à-dire la reproduction de documents transparents grâce aux diazoïques sensibles aux UV. Le ribose et les deux phosphates intercalés entre l'Adénine et le reste iso-alloxazine pourraient servir, quant à eux, d'électret de transfert luminescent entre les deux extrémités de la molécule.

Avant de préciser et développer l'intérêt de l'utilisation et l'adjonction de certaines vitamines et de principes actifs photo-actifs comme la vitamine B2 et le FAD, la vitamine PP et le NADP, lors de la fabrication d'articles protecteurs tels que les préservatifs et les gants à usage médico-chirurgical, il est apparu utile de résumer succintement le rôle de ces vitamines et principalement de la Riboflavine dans l'immuno-déficience humaine; les effets anti-viraux de la vitamine B2 ont été découverts en Novembre 1988 sur un malade sidéen -stade IV- et en Août 1989 sur un patient présentant une récurrence importante d'un Herpès génital contracté par voie sexuelle en 1970. Dans les deux cas, la vitamine B2 a été injectée par voie intra-veineuse à raison d'une injection unique de 20 mg par jour, avec des résultats spectaculaires chez ces deux patients. L'idée de départ découlait donc de constatations expérimentales concernant la photosynthèse protéique à l'échelle animale et la photosynthèse virale. On a donc utilisé initialement la vitamine B2 en fonction de ses propriétés d'absorption de la lumière avec l'idée d'interposer un écran photoprotecteur entre le rayonnement solaire UV parvenant à l'organisme et les virus HIV et HSV dont les patients étaient porteurs. Ce concept a été formulé dans le but d'"éteindre" ces virus dont il semblait, en fonction des hypothèses émises, que le rayonnement ultra-violet activait tant leur synthèse que leur replication. Les effets cliniques spectaculaires et très rapidement obtenus ont confirmé l'hypothèse de départ. C'est par la suite que le Demandeur s'est rendu compte qu'outre les propriétés photoprotectrices de la vitamine B2, la formule biochimique diphosphonucléotidique de son métabolite actif, le FAD, jouait également un rôle important.

Ces diverses constatations ont amené le demandeur à déposer une demande de brevet d'invention en FRANCE, le 2 Avril 1991 sous le numéro 91.04218).

Début 1992, un test "in vitro" a été effectué par l'Institut de Virologie de la Faculté de Strasbourg. Le test est positif à la dilution au 1/5000è sur les cellules CEM-SS infectées par HIV1; l'effet anti-viral est objectivé par une inhibition de la transcriptase reverse supérieure à 50%.

Au vu des effets "in vitro" de la vitamine B2 et en raison de la formule chimique diphosphonucléotidique du FAD, une demande de brevet internatinal, portant sur cette application, a été déposée le 2 Avril 1992 (PCT/FR92/00296). Des tests "in vitro" ont été réalisés et confirment que le FAD est aussi actif in vitro que l'AZT et plus actif que le DDI et ceci, sans aucune cyto-toxicité.

Durant la période allant de la constatation initiale à ce jour, les recherches du Demandeur ainsi que diverses déductions ont permis de reprendre globalement le processus de l'immunodéficience humaine tant dans son étiopathogénèse que son déroulement clinique et son traitement. Le couplage des théories photosynthétiques avec le rôle et l'influence des rayons UV puis des molécules photoprotectrices a permis de répondre, point par point, aux différentes interrogations et inconnues se rapportant au Sida. Un développement de ce raisonnement déductif est apparu indispensable afin de mieux comprendre le mode de propagation du virus HIV et l'intérêt de l'adjonction aux préservatifs, gants et doigtiers médico-chirurgicaux de certaines molécules ayant un rôle d'écran photoprotecteur, voire sono-protecteur comme la vitamine B2 et le FAD. Ces points principaux paraissent fondamentaux dans l'immunodéficience humaine et sont relatifs aux origines de l'infection virale, au temps de latence séparant le "contage" viral et au développement de la maladie, à la symptomatologie clinique observée et enfin aux cas récents de Sida sans virus, rapportés au récent congrès d'AMSTERDAM sur le Sida (1993). Les origines de l'infection sont vraisemblablement étroitement liées à l'environnement et à certains facteurs ethniques, géographiques, socio- économiques et enfin comportementaux individuels, voire génétiques. Au niveau de l'environnement, trois conditions principales ont entraîné une majoration de l'irradiation par les UV, couplée à une diminution de la filtration de ces mêmes UV, ce sont :
- la raréfaction de la couche d'ozone
- le traitement et la pollution des sols
- et enfin, l'exacerbation du cycle solaire et des explosions solaires.

La raréfaction de la couche d'ozone parait bien établie actuellement, son impact sur les individus aboutit, selon les connaissances actuelles, à une fréquence accrue des mélanomes et des cataractes et à la diminution des défenses immunitaires. Le mécanisme biologique intime des lésions observées parait en rapport avec une méthémoglobinémie plus fréquente et avec une augmentation des radicaux libres dont les conséquences sont bien connues code : l'anoxie tissulaire, la lipoperoxydation membranaire suivie parfois d'une atteinte génique consécutive à une irradiation exagérée de l'ADN.

Les recherches effectuées par le Demandeur sur la photosynthèse protéique à l'échelle animale, confirment et expliquent l'atteinte de la synthèse protéique et par là même, l'atteinte des immunoglobulines de défense. Il a été signalé, dans la carence en vitamine B2, une atteinte cutanéo-muqueuse, des cataractes et une diminution des défenses immunitaires. On sait aussi que lorsque l'on expose du lait, même dans un emballage transparent à la lumière, 85% de la Riboflavine sont détruits.

Les organismes humains similaires à des récipients de lait, vont épuiser progressivement leurs réserves tissulaires en vitamine B2 selon un phénomène compréhensible de surconsommation. Les nourrissons et les jeunes enfants ayant très peu de réserve tissulaire, résisteront moins longtemps à l'agressivité des UV. D'autre part, les mères carencées ne leur apportent pas suffisamment de vitamine B2 par l'allaitement.

L'atteinte cutanéo-muqueuse, liée à la carence en vitamine B2, touche notamment : la bouche, la vulve, l'anus, le scrotum et le pénis. Il est alors facile de comprendre la dissémination vénérienne qui s'ensuit.

Le traitement des sols par divers produits et molécules chimiques tels que pesticides, insecticides, engrais phosphatés, azotés parmi lesquels des phénols et benzènes halogénés, va aboutir à intensifier l'irradiation photonique UV et la production de nitrites venant augmenter la survenue de méthémoglobinémie signalée précedemment. L'accumulation de déchets toxiques, la deforestation et la sécheresse, en diminuant les systèmes tampons, aggravent encore l'irradiation UV. Il parait évident que tout comme pour le paludisme, les pays chauds et ensoleillés seront les plus touchés. Ceci est largement confirmé par les proportions de malades sidéens nettement prédominantes dans les pays équatoriaux et péri-équatoriaux. On remarque également que dans ces zones fortement ensoleillées, les végétaux agressés par les UV, les insecticides et les pesticides verront, comme les humains, leurs stocks vitaminiques et particulièrement de vitamine B2 amoindris d'où l'aggravation du déficit par carence d'apport au niveau des populations autochtones. Comme le même phénomène s'étend obligatoirement au bétail, il y aura encore majoration de la carence d'apport en vitamine B2 par le lait (oeuf, viande, céréales, fruits et légumes). Il faut signaler ici que si la vitamine B2 est préférentiellement citée, les autres systèmes tampons photoprotecteurs et anti-oxydants seront obligatoirement concernés et carencés par un triple phénomène lié à une carence progressive par suite d'une utilisation excessive, à une diminution des apports et enfin, à une malabsorption sous la dépendance des différentes pathologies générées et principalement les pathologies gastro-intestinales et hépatiques.

De nombreuses revues scientifiques ont fait état, depuis plusieurs années, d'un facteur aggravant l'irradiation UV excessive. Les explosions solaires paraissent considérablement augmenter tant en ce qui concerne leur fréquence que leur intensité.

L'étude de la prévalence du Sida par rapport aux éthnies soulève une remarque : c'est le parallélisme géographique fréquent existant entre paludisme et SIDA. Selon les théories photosynthétiques, le paludisme est chronologiquement antérieur au Sida car le paludisme ne serait, en fait, qu'une maladie de l'adaptation destinée à limiter la photocaptation exagérée des individus. D'ailleurs la présence de vitamine B2 ainsi que de nombreux autres agents photoprotecteurs au niveau des globules rouges parait confirmer cette hypothèse. Les proportions de sujets touchés en fonction des ethnies peut permettre d'établir une échelle des races par ordre décroissant de sensibilité : noire, indonésienne, blanche et enfin jaune. La peau parait jouer un rôle important dans l'immunodéficience, outre la protection qu'elle exerce par rapport à l'irradiation UV (mélanogénèse, carotène et vitamine A, vitamine E, cholestérol, vitamines B2 et PP). La peau possède un rôle immunitaire important en rapport avec les cellules de langherans et les mélanocytes. Il faut remarquer que les sujets de race noire malgré la mélanogénèse importante présentent des particularités au niveau de la mélanine. Le pigment est très lâche et présente une activité immunitaire lysosomiale très faible. L'ASIE est principalement touchée au niveau des zones indiennes et indonésiennes où la pigmentation parait beaucoup plus tirer sur le noir et le blanc que sur le jaune. Vient ensuite la race blanche. La race jaune pure, quant à elle, parait beaucoup moins touchée.

Ce fut au départ des recherches du Demandeur, un indice qui orienta vers le choix d'une molécule pigmentante jaune, en vue du traitement du Sida. De même que la tuberculose et d'autres grands fléaux, le Sida prédomine au niveau des populations défavorisées parmi lesquelles la malnutrition protéique est notoire. Or, il est bien connu qu'une alimentation carencée en protéines induit une fuite urinaire de la vitamine B2. On comprend alors que chez ces sujets, on voit se combiner une carence par excès d'utilisation (surconsommation), une carence d'apport avec carence par malabsorption et enfin, une carence par fuite urinaire. Privé d'agent photoprotecteur agissant comme les "lunettes de soleil", l'organisme de ces populations est la proie des UV. A ces différentes conditions de carence en vitamine B2 géographiques et ethniques, peuvent s'ajouter certains facteurs individuels en rapport avec des maladies, les effets intercurrents des médicaments, la toxicomanie, le stress et enfin la génétique. Il est connu que de nombreuses maladies amoindrissent à la fois le stock vitaminique et le système immunitaire. De nombreux remèdes présentent également un rôle inhibiteur sur les vitamines (antibiotiques, contraceptifs, anti-épileptiques, etc..). Le stress exacerbateur des systèmes catécholaminergiques, joue, en ce qui concerne les Européens un rôle aggravant majeur, puisque les neuromédiateurs concernés et mobilisés en excès, sont photo- attracteurs. Il en est de même pour de nombreuses drogues, alcool, cocaïne, héroïne, amphétamines qui, outre leurs noyaux photocapteurs, sont bien connues pour majorer le stress et détériorer le pool vitaminique et immunitaire des individus.

Le temps de latence séparant la primo-infection et la maladie sidéenne surprend de nombreux chercheurs. Il confirme à notre sens une bipolarité fondamentale de l'immunodéficience humaine selon laquelle l'effondrement des systèmes de défense est progressif. Le virus HIV n'est pas forcément l'élement déterminant de la maladie mais sans doute un témoin de son émergence, voire même dans un premier temps, un agent de défense et d'adaptation comme on le développera plus loin.

La pathogénèse du Sida parait reposer sur un triplet fondamental : une irradiation et une photocaptation UV exagérées, un épuisement progressif des systèmes tampon avec, pour aboutissement de ces deux conditions premières, un effondrement progressif de la synthèse des anticorps et par-là même du système immunitaire. Le demandeur a déjà signalé le phénomène initial princeps relatif à une irradiation UV excessive et la carence consécutive en vitamine B2 qui lui paraît représenter la clef de voûte du système photoprotecteur et anti-oxydant de l'organisme. A partir de cette carence en vitamine B2 et de celle de son métabolite actif le FAD, il va se produire, tel un jeu de dominos, une atteinte carentielle, en cascade, de nombreux autres agents enzymatiques et coenzymatiques de l'oxydo-réduction. Cette atteinte est en rapport avec à la fois une interrelation métabolique et biochimique liée essentiellement à l'effondrement enzymatique en rapport avec le système d'oxydo-réduction sous-tendu par le FAD et le NAD. L'atteinte carentielle des flavoprotéines intéresse l'organisme entier. La deuxième raison est que cette carence en flavoprotéines génère des symptômes digestifs en particulier intestinaux (diarrhées) et hépatiques venant provoquer une malabsorption de nombreuses autres vitamines. Enfin, le FAD et le NAD sont indispensables à la dégradation catabolique des aliments. Les maladies infectieuses opportunistes aggravent, par elles-mêmes, le déficit vitaminique de même que l'antibiothérapie itérative qu'elles nécessitent. L'addition des carences et de l'irradiation UV excessive entrainant un épuisement tissulaire vitaminique de la carence primaire en vitamine B2, va intéresser la chaîne respiratoire et le système tampon anti-oxydant, c'est-à-dire anti-radicalaire permettant de limiter en particulier la méthénoglobinémie.

Autant l'ADN, avec les chromosomes et les gènes, est à la base de la synthèse protéique, autant la chaîne respiratoire cellulaire est fondamentale pour le bon fonctionnement métabolique et énergétique de la cellule. La carence relative en Riboflavine et FAD va tout d'abord paralyser la chaîne respiratoire cellulaire composée essentiellement par deux transporteurs d'hydrogène : la vitamine B2 et la vitamine PP en continuité avec le système des cytochromes dont le rôle est le transfert des électrons. La jonction entre les transporteurs de protons et les transporteurs d'électrons est assurée par un système quinone-hydroquinone sous la dépendance du cytochrome Q, très proche de la vitamine E. La paralysie de la chaîne respiratoire contribue à inhiber le catabolisme énergétique et les synthèses cellulaires. Outre l'anoxie cellulaire et le déficit énergétique va se produire une accumulation de radicaux libres. Le déficit en vitamine B2 et FAD touche d'abord la vitamine PP et le système enzymatique qu'elle induit, et plus précisement, ses deux cofacteurs : le NAD et le NADP.

Le FAD est indispensable à la réoxydation de la NADH en NAD et du NADPH en NADP. Cette deuxième réaction catalyse l'activité de la glutathion réductase érythrocytaire.

La carence en vitamine B2 inhibe également la NADH méthémoglobineréductase érythrocytaire. Ceci confirme qu'outre la paralysie de très nombreuses réactions enzymatiques sous la dépendance des systèmes FAD et NAD, il va survenir une accumulation de radicaux libres et une augmentation de la méthémoglobinémie dont le rôle apparait fondamental dans la génèse de l'immunodéficience. La répartition dans l'organisme de la vitamine B2 et de la vitamine PP photoprotectrices, toujours en continuité, n'obéit pas à une loi du hasard. La démonstration de la photosynthèse protéique à l'échelle animale, confirme que ces deux vitamines, qui assurent un rôle de commutateur et de modulateur optique et très vraisemblablement ultra-sonique, sont situées au niveau des principaux filtres à UV de l'organisme. Une telle disposition permet ainsi de moduler les apports opto-électroniques optimaux parvenant aux membranes cellulaires, à l'ADN et à la moëlle osseuse. Les membranes cellulaires sont analogues par leur structure à des transistors et il parait logique de concevoir que la quantité de lumière et par là même d'électricité atteignant ces membranes, soit optimale afin de permettre l'ouverture alternative des canaux membranaires. De la même manière l'ADN, dont on connait l'affinité pour les UV, doit recevoir une dose optimale d'énergie, afin de satisfaire physiologiquement ses fonctions de synthèse protéique ou de division cellulaire. La moëlle osseuse quant à elle est très sensible à l'irradiation qui peut dégrader définitivement une lignée cellulaire. La production de 800 milliards de cellules par jour nécessite un impact opto-électronique et photonique très précis, afin de respecter les synthèses sans nuire aux cellules souches.

Après avoir touché la vitamine PP, la cascade carentielle atteint l'acide folique. C'est ainsi que l'inhibition du système enzymatique FAD-dépendant empêche la réduction de l'acide folique en sa forme circulante active : le méthyltétrahydrofolate.

Les réactions biochimiques tissulaires effectuées à partir de la transformation du méthyltétrahydrofolate sont ainsi inhibées. C'est ainsi qu'on s'intéressera plus particulièrement à l'inhibition de la synthèse des bases puriques à partir de l'acide inosinique, à l'inhibition de la synthèse des bases pyrimidiques et de l'acide desoxy-thymidylique. Ceci est particulièrement intéressant car on peut se demander si la phosphorylation de certaines bases puriques et pyrimidiques ne constituerait pas "in vivo" des équivalents endogènes de remèdes comme l'AZT, la DDI et la DDC.

Donc, l'inhibition des synthèses puriques et pyridimiques en rapport avec une carence en vitamine B2 et en FAD annihilerait un système biologique de défense endogène anti-viral et plus particulièrement anti-VIH.

Un deuxième phénomène intervenant au niveau de l'acide folique retiendra particulièrement l'attention. Sous l'effet d'une irradiation excessive UV, il va y avoir une carence relative en vitamine B2 et en FAD et une absence de transformation par réduction, de l'acide folique en méthyltétrahydrofolate. L'acide folique non transformé est alors scindé par l'irradiation UV excessive au niveau des carbones 9-10 avec libération d'acide para-amino-benzoïque dont on connait le rôle catalyseur au niveau des synthèses bactériennes. On comprendra donc mieux le pourquoi de nombreuses infections opportunistes rencontrées au cours du Sida. Enfin, l'acide folique intervient au niveau du métabolisme de trois acides aminés importants : l'acide glutamique, la cystéine et le glycocolle. Lorsqu' ils sont associés, ces trois acides aminés constituent la molécule de glutathion qui joue un rôle majeur dans la respiration cellulaire, en particulier grâce au FAD et au NADPH, qui permettent le fonctionnement de la glutathion-reductase érythrocytaire. La carence des systèmes réducteurs NADPH et FADH2 inhibe la glutation-réductase et le système tampon radicalaire glutathion peroxydase/glutathion réductase.

Dans ces conditions, le glutathion ne peut plus jouer son rôle capital de commutateur optique et d'échangeur de radicaux libres. En conséquence, la modulation des UV n'étant plus assurée, la production de radicaux libres et de péroxydes augmente et cela d'autant plus que les systèmes tampon, en rapport avec le FAD, le NAD et le glutathion, sont inhibés.

Il est connu que le glutathion exerce une activité anti-VIH in vitro. Il est permis de supposer que son inactivation, par défaut de FAD, de NAD et d'acide folique, peut contribuer au développement du VIH in vivo, par voie de conséquence.

L'acide folique intervient également avec les vitamines B2, B12, B6 et C dans la synthèse de l'Hème. Comme le glutathion, l'Hème exerce une activité anti-VIH in vitro. Une diminution de sa biogénèse pourrait-être aussi propice au développement du VIH in vivo et ceci, en raison de la carence en cytochromes d'origine héminique et particulièrment, les cytochromes de la chaine respiratoire cellulaire et le cytochrome P450 qui joue un rôle majeur dans la détoxication hépatique, également sous la dépendance du NADPH. Ainsi, cette détoxication hépatique intéresse non seulement les médicaments mais également le métabolisme de certaines substances endogènes comme la bilirubine et les hormones.

L'acide folique intervient enfin dans la synthèse des acides biliaires par l'intermédiaire du glycocolle et de la taurine qui provient de la cystéine. Comme le glutathion et l'hème, les acides biliaires sont actifs in vitro sur le VIH et une atteinte du métabolisme biliaire, au niveau de la bilirubine et des acides biliaires, favoriserait ainsi le développement du VIH in vivo et contribuerait à aggraver les carences de certaines vitamines code la vitamine E et la vitamine A dont l'absorption est sous la dépendance de la bile.

On voit donc l'importance que peut présenter, par rapport à l'immunodéficience humaine, une photo-irradiation UV exagérée avec atteinte des vitamines B2, PP et acide folique : il va se produire une inhibition de nombreux systèmes enzymatiques de la chaine respiratoire cellulaire et des systèmes tampons anti-radicalaires qui va générer des lésions cellulaires protéiformes avec diminution de la synthèse des anticorps et aussi des systèmes de défense endogènes anti-VIH.

Comme on l'a décrit plus haut, la cascade carentielle touche la vitamine E dont le rôle photoprotecteur et anti-oxydant est très important tant au niveau de la chaîne respiratoire cellulaire que du système tampon glutathionique.

La vitamine C peut être touchée également par malabsorption en raison des troubles digestifs et des diarrhées provoqués par la carence en vitamines B2, PP et en acide folique.

Différentes agressions, telles que le stress, les drogues, les infections opportunistes et les antibiothérapies qui leurs sont afférentes, vont aggraver la carence en vitamine C par l'augmentation des besoins. Le rôle anti-oxydant majeur, joué par la vitamine C au niveau de la respiration cellulaire ainsi que son rôle immunologique très important, contribuent à expliquer la propagation de l'infection à VIH. A l'instar des vitamines C et E, la vitamine A et les caroténoides jouent un rôle important dans l'immunodéficience et la photo-protection. Celle-ci s'exerce au niveau de l'oeil, des épithéliums, du tissu adipeux et des membranes cellulaires. Le rôle anti-infectieux et anti-cancéreux de la vitamine A est vraisemblablement en relation avec ses effets photoprotecteurs importants. L'organisme est susceptible d'être carencé en vitamine A, non seulement par les diarrhées qui entrainent sa malabsorption mais encore par l'atteinte hépatique en rapport avec la carence en vitamine B2 puis l'immunodéficience. Le métabolisme de la vitamine A est, par ailleurs, étroitement lié aux systèmes enzymatiques FAD et NAD-dépendants. L'atteinte de ces systèmes aboutit ainsi à des troubles de l'utilisation périphérique de la vitamine A et des carotènes.

Enfin, la vitamine A ne peut être correctement transportée à des sites actifs puisque la protéine (la rétinol-binding-protéine) qui assure son transport plasmatique, est vraisemblablement réduite jusqu'à la carence en raison de l'atteinte hépatique et de l'inhibition de la synthèse protéique.

Une équipe médicale d'AFRIQUE DU SUD a signalé récemment une amélioration notable de la rougeole à la suite de traitement par la vitamine A. Il parait probable que c'est par son rôle photoprotecteur que la vitamine A a pu inhiber le virus morbilliforme dont on sait qu'il est beaucoup plus virulent dans les pays chauds.

La cascade carentielle touche encore la vitamine B6 dont l'action couplée avec la vitamine PP vis-à-vis du tryptophane et de la sérotonine en fait un puissant modulateur de lumière. La vitamine B6 catalyse la transformation du tryptophane en NAD et vitamine PP, par la voie de la Cynurénine, c'est-à-dire qu'un excès de lumière au niveau de l'organisme module sa photocaptation en orientant son métabolisme vers des molécules photoprotectrices. Il a été signalé, dans la carence humaine en vitamine B6 lorsqu'elle se prolonge, une lymphopénie. Celle-ci parait en rapport avec une irradiation UV excessive. L'atteinte du système lymphatique et des lymphocytes chez les sidéens parait donc bien en rapport avec un excès d'irradiation UV. Cette hypothèse est corroborée par le taux des lymphomes relativement important chez les sidéens. Des expériences récentes de photophérèse ou plasmaphérèse après sensibilisation au 8-méthoxy-psoralène et irradiation UV confirment, sans conteste, les rapports qui paraissent exister entre lymphomes et UV. Selon les reflexions exprimées ci-dessus, l'atteinte lymphocytaire qui joue un rôle majeur dans l'imunodéficience serait en rapport avec un phénomène de ciblage des lymphocytes T4 par le HIV.

Les glycoprotéines virales et aussi l'interféron de nature lui-même glycoprotéique, produit par le virus au niveau des lymphocytes T4, vont exciter la photocaptation par les lymphocytes lors de leur passage dermique et surtout thymique.

On sait, en effet, que la région cervicale antérieure, avec la thyroïde et le thymus, est très sensible aux irradiations. Les lymphocytes T4, sensibilisés par le HIV et les glycoprotéines, seraient ainsi marqués ou ciblés puis enfin détruits par un "tir au laser". Le ciblage laser a été remarqué lors de la guerre du Golfe. Les avions équipés du ciblage laser frappent ainsi leur objectif au millimètre près. Ce phénomène de ciblage et de destruction par la lumière peut paraître surprenant pour des biologistes ou biochimistes.

On peut préciser ici le sens de la pensée du demandeur en expliquant le phénomène de la vitesse de sédimentation. Lors des maladies inflammatoires, la VS est accélérée, c'est-à-dire qu'après centrifugation, la sédimentation des globules rouges est beaucoup plus rapide. La biochimie ne peut expliquer le phénomène intime préludant à cette accélération.

Les considérations précédentes, sur la photosynthèse animale, révèlent que tout d'abord les protéines de l'inflammation sont spiralaires et polarisées à leurs deux extrémités. Elles correspondent donc à un accumulateur chercheur et à un solénoïde. Le champ lumineux, et par-là même électrique et magnétique, qui les traverse, est donc considérablement intensifié et accéléré.

Dans ces conditions, l'amplification du rayonnement à travers les protéines inflammatoires va accélerer la sédimentation des globules rouges puisqu'ils captent un faisceau électro-magnétique beaucoup plus puissant que celui qui traverse un sérum normal.

On vient de voir comment à partir d'une irradiation UV excessive en rapport avec l'environnement "couche d'ozone", la situation géographique des régions les plus touchées, la malnutrition protéique et certains facteurs personnels comme le stress, les toxicomanies, les maladies et certains médicaments, la vitamine B2 peut être relativement carencée par utilisation excessive. Il peut s'y rajouter une diminution des apports, voire une malabsorption. Il en résulte un épuisement progressif des réserves tissulaires.

La carence en vitamine B2 couplée à l'irradiation excessive, touche ensuite la vitamine PP, l'acide Folique, la vitamine E, la vitamine A, la vitamine B6 et les caroténoïdes qui nous paraissent tous jouer un rôle important anti-oxydant et photoprotecteur. Cette cascade carentielle vitaminique peut intéresser, en outre, d'autres vitamines comme la vitamine B12, la biotine, l'acide pantothénique et le CoA. Les interrelations vitaminiques laissent supposer que tout le système vitaminique est ainsi touché. Les conséquences en sont plus que préjudiciables à tous les organismes concernés eu égard au rôle carrefour représenté par les vitamines qui sont des facteurs indispensables à la vie comme leur nom l'indique.

Les principales conséquences sont initialement métaboliques avec une dégradation des processus cataboliques et énergétiques de la chaîne respiratoire cellulaire, des synthèses cellulaires et du système immunitaire. Les neuro-médiateurs et les hormones étroitement dépendants des vitamines sont également touchés tant dans leurs synthèses que dans leur fonctionnement biologique.

On a déjà évoqué comment la diminution progressive de nombreux systèmes enzymatiques et coenzymatiques avec leurs conséquences en particulier immunitaires, permettent d'expliquer le temps de latence variable lors de l'immunodéficience humaine. L'atteinte primaire de la Riboflavine et par suite de la chaîne respiratoire cellulaire, associée à l'effondrement progressif de la synthèse des immunoglobulines, permet de mieux comprendre la dualité Sida-cancer.

On peut ainsi préciser comment la carence en vitamine B2 peut expliquer les formes sans virus récemment décrites au Congrès d'AMSTERDAM de Sida.

Les patients atteints de telles formes à évolution mortelle sont porteurs d'anticorps anti-VIH alors que le virus ne peut être identifié au niveau des organismes malades. La carence en vitamine B2 s'accompagne, au fil de son évolution, d'une acidose organique sévère qui est d'ailleurs utilisée comme test diagnostic d'approche de la carence en vitamine B2. Cette acidose organique est tissulaire, plasmatique et urinaire.

Les manuels spécialisés apprennent que les membranes virales sont coaptées grâce à des forces de tension. Il parait presque évident que ces forces de tension sont en fait assimilables à un phénomène d'attraction électromagnétique en rapport avec le Calcium++, le Magnésium++ et le Fer++. Ces métaux sont très sensibles à l'acidose et à l'oxydation, qui en les dissolvant ou les transformant en Fer ferrique vont ainsi annihiler le phénomène d'aimantation membranaire électromagnétique. La physique quantique permettrait même d'introduire des notions de Diamagnétisme et Paramagnétisme. Celles-ci en fait, ne sont pas indispensables à la compréhension de la dissolution virale.

En conclusion, on peut admettre que dans certaines conditions d'acidose majeure en rapport avec la carence en Riboflavine, les virus présents au début de la maladie, initient une synthèse spécifique d'anticorps anti-VIH, et sont dans un deuxième temps dissouts par l'acidose. Cette dissolution n'empêche pas la poursuite de la dégradation des systèmes immunitaires en rapport avec la cascade carentielle vitaminique exponentiellement croissante, citée dans ce qui précède.

Si l'on conçoit que le virus peut au départ être un agent opto-électronique de défense de l'organisme comme on le verra plus loin, on comprendra alors que la lyse virale en l'absence du rétablissement des messages opto-électroniques biologiques va être très préjudiciable à l'organisme et faire ainsi de ces formes sans virus des formes éminemment graves.

Cet exposé, à propos de la vitamine B2, des UV et du HIV parait cependant nécessaire afin de mieux comprendre le bénéfice que l'on pourrait tirer de l'adjonction de la vitamine B2 à la structure de base des préservatifs, doigtiers et gants médico-chirurgicaux.

Ce bénéfice qui apparaît fort important dépend principalement de certains symptômes décrits dans la carence en Riboflavine et de l'aspect électro-physique du virus HIV.

Les manuels se référant aux vitamines signalent lors de la carence en vitamine B2, deux constatations qui ont une importance très grande dans le sens qu'elles confirment les hypothèses concernant l'origine du Sida, l'action des UV et le rôle de la vitamine B2.

La carence en vitamine B2 entraine tout d'abord l'apparition de lésions cutanéo-muqueuses, bucco-pharynghées et ano-génitales. Ces lésions consistent en inflammation, desquamation voire fissuration et s'accompagnent parfois d'un prurit sévère touchant particulièrement les régions anales, vulvaires, péniennes et scrotales. Il est alors facile de comprendre comment les sujets carencés en vitamine B2 sont particulièrement concernés par une contagion "contage" ou une transmission virale oro-génitale. Le deuxième élément clinique qui nous parait capital est représenté par la constatation d'une hyperpigmentation de la vulve et du scrotum chez les sujets carencés en vitamine B2.

L'explication d'un tel phénomène qui vient corroborer les rapports existants entre virus, vitamine B2 et photocaptation parait simple à expliquer : la carence en vitamine B2 photoprotectrice initie un phénomène d'adaptation de défense compétitif. Une mélanogénèse accentuée au niveau des organes génitaux vient compenser la carence en Riboflavine de ces régions dont on connait la grande fragilité vis-à-vis de l'irradiation.

On conçoit, à la lumière de ces deux observations, le rôle majeur préventif, curatif et protecteur de la vitamine B2 utilisée tant par voie générale que par voie locale.

L'étude des virus HIV et HSV sous leur aspect électrophysique renforce le souci d'augmenter les effets isolants des préservatifs et gants de protection. La vitamine B2 et son métabolite actif le FAD, en conférant aux matériaux protecteurs une coloration jaune-orangée, va permettre une opacification, une isolation vis-à-vis d'une transmission sonique et opto-électronique. Une telle affirmation pourrait paraître surprenante à l'échelle macroscopique. Son explication est en vérité du domaine des nano-techniques.

Les études, sur la photosynthèse protéique et la photosynthèse virale, ont habitué depuis plusieurs années à raisonner au nivau de l'infiniment petit. L'explication de la messagerie opto-électronique, au niveau histo-physiologique et biologique, nécessitait d'établir une jonction étroite entre les microstructures organiques et les applications industrielles des nano-technologies en particulier dans les domaines informatiques et vidéo.

Le virus est ainsi apparu représenter un récepteur-émetteur à modulation de fréquence pouvant servir d'antenne-relais au niveau cellulaire. Si dans certaines conditions pathologiques l'ADN cellulaire ne peut plus recevoir un message éléctronique adéquat, cela peut entraîner une distorsion des synthèses protéiques avec production de protéines anormales parmi lesquelles vont se glisser des erreurs de transcription pouvant toucher l'agencement normal des acides aminés ou même modifier certains acides aminés des chaines peptidiques, remplacés par d'autres acides aminés avec toutes les conséquences que cela peut ainsi entrainer tant à l'échelle de la cellule que de l'organisme entier. L'approche cellulaire par opto-electronique qui nous a permis de redéfinir un code génétique non dégénéré à 28 acides aminés permet, en effet, de comprendre comment les gènes messagés par des impacts électroniques se comportent comme une machine à écrire.

Ce mode de raisonnement et d'explication de la biologie cellulaire et de la génétique permet de comprendre la génèse des maladies génétiques bien évidemment différentes en fonction des gènes concernés. De telles hypothèses sont d'ailleurs coroborées par le rôle de phages d'origine virale qui sont indispensables au bon fonctionnement de l'ADN bactérien.

Une façon élégante de résoudre le problème est d'incorporer à cette cellule un module qui servirait à la fois d'antiparasite et de transistor FM relais. Un tel poste de secours pourrait ainsi, dans un premier temps, rétablir le passage du message physiologique par son rôle d'antiparasite puis dans un deuxième temps servir de relais dépanneur à l'ADN. Les virus paraissent tout à fait habilités à exercer de telles fonctions et plus précisemment le virus HIV. La structure stéreo-physique des virus confirme amplement leur rôle et fonction d'appareils recepteurs-émetteurs à modulation de fréquence.

Les spicules glyco-protéiques peuvent être des antennes réceptrices puis émettrices, l'ADN des rétrovirus représentant l'organe de captation, analyse puis programmation. On rappellera encore une fois que l'opto-élecronique peut tout aussi bien transmettre des sons -la téléphonie moderne est électronique- que des images numériques vectorisées par des électrons. La taille des virus qui est de quelques dizaines de nanomètres correspond donc à des dimensions de l'ordre de 10⁻⁸m. C'est-à-dire que les virus peuvent entrer en phase avec lla longueur d'onde de la lumière ultra-violette. La dimension des virus permet ainsi de comprendre comment, grâce à la parfaite coaptation de phase avec les UV, sera générée une résonnance optimale.

Les hypothèses sur le rôle électro-physique des virus sont pleinement confortées par le rapport du Docteur S.WAIN-HOBSON. Ce rapport relate la découverte de formes non virulentes du virus HIV au GABON et la mise en évidence d'une modification dans la séquence du gêne tate du virus; au niveau de ce gène, il y a remplacement d'un codon destiné à la cystéine par un codon destiné à la sérine. la cystéine à l'inverse de la cytrine est une molécule chromophore.

L'aspect électrophysique du virus HIV permet aussi de proposer une explication concernant la mort des cellules porteuses. Le virus inclus dans la cellule reçoit un voltage optimum. Lorsque le seuil de captation est dépassé à la suite de diverses conditions comme une irradiation UV majeure, un stress, une agression en rapport avec la toxicomanie ou enfin, une carence accrue des systèmes tampons et photoprotecteurs, le virus peut alors imploser en même temps que la cellule. Nous avons vu précedemment comment les glycoprotéines virales photo-attractrices peuvent cibler les cellules porteuses. Il est alors facile de concevoir comment un éclair lumineux plus intense peut entrainer un survoltage brutal et par là même, l'explosion de la cellule ciblée.

Ces diférentes considérations sur la vitamine B2 et le VIH confirment une fois encore l'intérêt d'utiliser la vitamine B2 et le FAD au niveau des préservatifs, doigtiers et gants médico-chirurgicaux où ces deux molécules pourront jouer un rôle de paravent opto-électronique ainsi que biochimique.

La réalisation technique consistant à inclure la vitamine B2 et le FAD au niveau du latex a amené à l'élaboration et la possibilité d'une triple méthodologie faisant appel à des procédés d'inclusion, d'interposition ou de recouvrement. Le procédé d'inclusion parait tout à fait réalisable à condition de respecter certaines conditions physico- chimiques. Il consiste à mélanger la vitamine B2 et le FAD à l'un des bains de préparation du latex afin de pigmenter celui-ci tout en ne nuisant pas à la structure biochimique des composés pigmentants.

La vitamine B2 étant inactivée par les UV lorsqu'elle est en solution alcaline, il est opportun de prévoir un bain légèrement acide. L'acide ascorbique dont on connait les propriétés anti-oxydantes et antivirales, peut constituer un adjuvant acide de choix. Par contre, si la vitamine B2 et le FAD sont sensibles à la lumière, elles sont thermostables, ce qui ne devrait donc pas compromettre l'intégrité de leur structure lors de la vulcanisation à chaud du latex.

Le premier procédé d'inclusion impose donc trois précautions principales : il faut tout d'abord éviter une trop forte luminescence au niveau des cuves de préparation du latex; secondement, il sera préférable d'utiliser si possible, un bain légèrement acide puis troisièmement, tester la compatiblité entre la vitamine B2, le FAD et les nombreux adjuvants nécessaires à la préparation du latex.

Le deuxième procédé selon l'invention concerne essentiellement la réalisation de préservatifs en latex bi-couches, ce qui laisse la possibilité d'interposer entre les deux couches de latex une solution de vitamine B2, de FAD et éventuellement de vitamine C. Ce procédé est également applicable aux gants et doigtiers.

Le troisième procédé dit de recouvrement, consiste à adjoindre lors de la présentation de préservatifs simples et translucides, voire de préservatifs fabriqués selon les méthodes 1 et 2, une crème ou un gel lubrifiant de couleur jaune-orangée, conférée par la vitamine B2, et le FAD. Il sera possible d'adjoindre d'autres vitamines précédemment citées comme la vitamine E, la vitamine A et les carotènes, l'acide folique, la vitamine C et aussi la vitamine PP et le NADP. Ce gel est destiné à s'appliquer au contact de la peau et des muqueuses puis sur le préservatif.

Les procédés définis 1, 2 et 3 concernent des objets protecteurs en latex qui peuvent s'utiliser de manière séparée ou à l'inverse, conjointement. Le talc est déconseillé en raison de ses propriétés irritantes.

L'évolution des techniques des bio-matériaux ainsi que certains cas de sensibilisation grave au latex laissent entrevoir l'utilisation de matériaux polymériques de base, différents. L'adjonction de vitamine B2 et de FAD voire d'autres vitamines protectrices à ces matériaux, est tout à fait envisageable comme pour le latex, et fait partie de l'invention.

Des gants et doigtiers en chlorure de polyvinyle existent déjà sur le marché. Comme pour le latex, les techniques d'inclusion ou de recouvrement peuvent s'appliquer aux articles mono-couches et la technique d'interposition quant à elle, utilisée isolement ou couplée aux deux autres procédés, peut s'appliquer aux articles bi-couches.

Il est enfin possible d'envisager d'étendre l'utilisation de la vitamine B2 et du FAD et des vitamines déjà citées à d'autres objets de protection tels que les masques, calots, gaze, coton, blouses, draps ou alèses afin de protéger au mieux du risque de contamination virale, en particulier dans les services hospitaliers accueillant des malades sidéens, quelque soit le matériau utilisé.

### EXEMPLE

On prépare dans une cuve chauffée à double enceinte 100 l d'une émulsion de latex à 10% dans l'eau comportant un agent assouplissant comme un polyéthylène glycol 4000 ou 6000 et un agent antivieillissement comme le terbutyl paracresol ou le diterbutyl paracrésol. On y incorpore, sous agitation, 1% d'un agent de glissement comme le stéarate de Calcium. A cette émulsion blanchâtre, on ajoute progressivement, sans cesser l'agitation, 100 ml d'une solution à 0,05% de chlorhydrate de riboflavine et à 0,2% de flavine -adenine dinucleotide (FAD).

Lorsque le mélange est complètement homogène, on verse l'émulsion dans des moules et on procède à la coagulation à chaud. Après calandrage à 80°, on ouvre les moules, on laisse refroidir, on lave à l'eau les gants et on en lubrifie l'intérieur. Les gants sont séchés sous courant d'air et répartis dans des alvéoles. Les gants ainsi préparés sont imprégnés de riboflavine et de FAD.

## Revendications

1. Articles d'hygiène en latex ou en chlorure de polyvinyle caractérisés en ce que l'on incorpore dans la masse du matériau polymérique de la riboflavine ou un de ses sels et de la Flavine Ademine Dinucleotide en quantité efficace pour jouer un rôle de "paravent" opto-électronique et biochimique.

2. Articles d'hygiène selon la revendication 1. constituées par des gants médico-chirurgicaux.

3. Articles d'hygiène selon la revendication 1. constitués par des doigtiers médicaux.

4. Articles d'hygiène selon la revendication 1. caractérisés en ce qu'il s'agit de préservatifs.

5. Un procédé d'obtention des articles d'hygiène selon la revendication 1. qui consiste à mélanger la riboflavine ou à un de ses sels et le FAD à un des bains de préparation de latex en milieu légèrement acide, afin d'incorporer la riboflavine et le FAD dans la masse de polymère éventuellement additionnée d'adjuvant sans nuire à la structure biochimique des composants colorants, à effectuer le moulage des articles d'hygiène à chaud, puis à procéder à une vulcanisation puis après refroidissement un rinçage à l'eau et au séchage.

6. Un procédé d'obtention des articles d'hygiène selon la revendication 1. caractérisé en ce que pour préparer des préservatifs en latex bi-couches ou interposer entre les deux couches de latex avant moulage une solution de Riboflavine ou d'un de ses sels, de FAD et éventuellement d'acide ascorbique.

7. Un procédé pour préparer les articles d'hygiène selon la revendication 1. caractérisé en ce que l'on adjoint aux préservatifs préparés selon le procédé de la revendication 5. ou de la revendication 6. une crème ou un gel lubrifiant de couleur jaune-orangé renfermant de la riboflavine ou un de ses sels et du FAD dont on tapisse la partie interne.

8. Un procédé selon la revendication 7. caractérisé en ce que la crème ou le gel lubrifiant contient, en outre, de la vitamine E et/ou de la vitamine A et/ou de l'acide folique et/ou de l'acide ascorbique et/ou de la vitamine PP et/ou du NADP.

9. Un procédé selon l'une des revendications 5 à 8. appliqué à des articles en gaz ou en coton comme des masques, des calots, des blouses, des draps ou des alèzes.

## Claims

1. Hygiene articles made of latex or polyvinyle chloride wherein into the mass of the polymeric material, they are incorporated riboflavin or a salt thereof and adenine dinucleotide flavine (ADF) in an amount effective for acting as an optoelectronic and biochemical "screen".

2. Hygiene articles according to claim 1 which are medico-surgical gloves.

3. Hygiene articles according to claim 1 which are medical fingerstalls.

4. Hygiene articles according to claim 1 wherein it is condoms.

5. A process for manufacturing hygiene articles according to claim 1 which consists in mixing riboflavin or a salt thereof and ADF with one of the preparation baths of latex in a slightly acid medium, in order to introduce riboflavin and ADF into the polymer mass wherein an adjuvant is optionally added without damaging the biochemical structure of the colouring matters, in making hygiene articles hot-molding, next in performing a vulcanizing step then after cooling a rinsing with water and drying.

6. A process for manufacturing hygiene articles according to claim 1 wherein for preparing condoms in latex with two layers between two layers of latex before casting out a solution of riboflavin or a salt thereof, of ADF or eventually of ascorbic acid.

7. A process for manufacturing hygiene articles according to claim 1 wherein it is added to condoms prepared according to the process of claims 5 or 6, a cream or an orange-yellow lubricating gel containing riboflavin or a salt thereof, and ADF with which the internal wall is covered.

8. The process according to claim 7 wherein that cream or lubricating gel further contains, vitamin E and/or vitamin A and/or folic acid and/or ascorbic acid and/or vitamin PP and/or NADP.

9. The process according to one of claims 5 to 8 applied to articles of gauze or cotton such as masks, caps, coats, sheets or undersheets.

## Patentansprüche

1. Hygieneartikeln aus Latex oder Polyvinylchlorid dadurchgekennzeichnet daß man in der Masse des polymere Material Riboflavin oder seiner Salze oder das Flavin-Adenin Dinucleotid, in einer Menge wirksame um eine Rolle von optoelectronische und biochemische Schirm zu spielen, einbringt.

2. Hygieneartikeln nach Anspruch 1., die aus ärztlisch-chirurgische Handschuhe bestehen.

3. Hygieneartikeln nach Anspruch 1., die aus ärztlische Fingerlinge bestehen.

4. Hygieneartikeln nach Anspruch 1., dadurch gekennzeichet daß es um einem Kondom handelt.

5. Verfahren zur Herstellung Hygieneartikeln nach Anspruch 1., das auf der Mischung des Riboflavins, oder einer seiner Salze oder des FAD mit eine der Herstellungsbäder von Latex in schwach säure Medium, um das Riboflavin und das FAD in der Masse des gegebenenfalls mit Hilfstoffen vermischte Polymers zu einbringen, ohne die biochemische Struktur des Farbstoffkomponente zu beschädigen, auf der Durchführung der Formung der Hygieneartikeln, denn auf dem Vorgang einer Vulkanisierung, denn auf nach Abkühlung, auf einer Spülung mit Wasser und dem Trocknen, beruht.

6. Verfahren zur Herstellung Hygieneartikeln nach Anspruch 1.,
dadurch gekennzeichnet daß man für die Herstellung von Kondome aus zweischichte Latex zwischen beider Latexschichte eine Lösung von Riboflavin vor der Formung zwischensetzt.

7. Verfahren zur Herstellung Hygieneartikeln nach Anspruch 1.,
dadurch gekennzeichnet daß man nach dem Verfahren der Anspruch 5 oder 6, hergestellten Kondomen eine Salbe oder eine schmierfähige Gel von gelb-orange Farbe zugibt, der die Riboflavin oder ein seiner Salze enthält, womit die interne Wall beschischtet wird.

8. Verfahren nach Anspruch 7., dadurch gekennzeichnet daß die Salbe oder der schmierfähige Gel weiterhein Vit E, und/oder Vitamin A, und/oder Folsaüre und/oder Ascorbin-saüre und/oder Vitamin PP und/oder NADP enthält.

9. Verfahren nach einer der Ansprüche 5 bis 8 das zu Artikeln aus Gab oder Baumwolle angewandt wird wie Gesichtsverband, Käppchen, Jacke, Tüche oder Undertüche.
